# EUROPEAN PATENT APPLICATION

(11) **EP 2 258 271 A1**
(43) Date of publication of application: **08.12.2010**
(21) Application number: 10151371.1
(22) Date of filing: 22.01.2010
(51) Int. Cl.: A61B 8/00, A61B 8/08, A61B 19/00

(54) **Comparison of ultrasound data before and after medical treatment**

(30) Priority: 01.06.2009 KR 20090048200
(71) Applicant: MEDISON CO., LTD., Kangwon-do 250-870 (KR)
(72) Inventor: Lee, Jin Yong, 135-851, Seoul (KR); Song, Young Seuk, 135-851, Seoul (KR); Kim, Jae Gyoung, 135-851, Seoul (KR)
(74) Representative: Schmid, Wolfgang

(57) **Abstract**

A system and a method of comparing ultrasound data associated with an object of interest before and after the medical treatment and providing the comparison information are disclosed. A plurality of first ultrasound data associated with an object of interest are stored before the medical treatment on the object of interest in the target body and a plurality of second ultrasound data associated with an object of interest are stored after the medical treatment on the object of interest in the target body in a storage unit. Instructions are input from a user for selecting any pair of first ultrasound data and second ultrasound data among a plurality of the first ultrasound data and a plurality of the second ultrasound data, which are stored in the storage unit. From the storage unit, the first ultrasound data and the second ultrasound data are extracted, and the extracted first ultrasound data and the extracted second ultrasound data are compared to form comparison information.

## Description

### CROSS-REFERENCE TO RELATED APPLICATIONS

The present application claims priority from Korean Patent Application No. 10-2009-0048200 filed on June 1, 2009, the entire subject matter of which is incorporated herein by reference.

### TECHNICAL FIELD

The present invention generally relates to an ultrasound system for comparing ultrasound data for a patient before and after a medical treatment. More particularly, the present invention relates to an ultrasound system capable of comparing ultrasound data for a patient, which are acquired at different statuses of the patient.

### BACKGROUND

An ultrasound system has been extensively used for acquiring information inside a target body due to its non-invasive and non-destructive nature. The ultrasound system has become an important tool in the medical field since it can provide doctors with high-resolution images of internal features of humans in real time without any surgical medical treatment.

Generally, the ultrasound system may acquire ultrasound data associated with an object of interest by transmitting ultrasound signals to the target body and receiving ultrasound echo signals reflected from the target body. The ultrasound system may provide an ultrasound image and/or a variety of information by using the acquired ultrasound data. For example, if the object of interest is a heart (or heart muscle), then the ultrasound system may provide dyssynchronous segment information for the analysis of strain, velocity, segment volume and the like by using the acquired ultrasound data.

Conventionally, ultrasound data associated with an object of interest obtained without medical treatment or ultrasound data associated with the object of interest obtained with medical treatment are used for providing dyssynchronous segment information. Thus, it is difficult to compare the degree of medical improvement before and after the medical treatment.

### SUMMARY

There are provided an ultrasound system and a method of comparing ultrasound data for a patient before and after medical treatment.

According to one aspect of the present invention, an ultrasound system comprises: an ultrasound data acquisition unit configured to acquire a plurality of first ultrasound data for an object of interest in a target body when the object of interest is in a first state and acquire a plurality of second ultrasound data for the object of interest when the object of interest is in a second state; a storage unit for storing the plurality of first ultrasound data and the plurality of second ultrasound data; an input unit configured to receive a user instruction for selecting at least one of the plurality of first ultrasound data and at least one of the plurality of second ultrasound data; and a processing unit configured to extract the at least one of the plurality of first ultrasound data and the at least one of the plurality of second ultrasound data from the storage unit and compare the extracted first ultrasound data with the extracted second ultrasound data to form comparison information.

According to one aspect of the present invention, there is disclosed a method of providing comparison information, which comprises: a) acquiring a plurality of first ultrasound data for an object of interest in a target body when the object of interest is in a first state and a plurality of second ultrasound data for the object of interest in the target body when the object of interest is in a second state; b) storing in a storage unit the plurality of first ultrasound data and the plurality of second ultrasound data; c) receiving a user instruction for selecting at least one of the plurality of first ultrasound data and at least one of the plurality of second ultrasound data; d) extracting the at least one of the plurality of first ultrasound data and the at least one of the plurality of second ultrasound data from the storage unit; and e) comparing the extracted first ultrasound data with the extracted second ultrasound data to form comparison information.

The summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- Fig. 1: is a block diagram illustrating an arrangement of an ultrasound system according to one embodiment of the present invention.
- Fig. 2: is a block diagram illustrating an arrangement of an ultrasound data acquiring unit according to one embodiment of the present invention.
- Fig. 3: illustrates exemplary ultrasound data registered according to one embodiment of the present invention.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

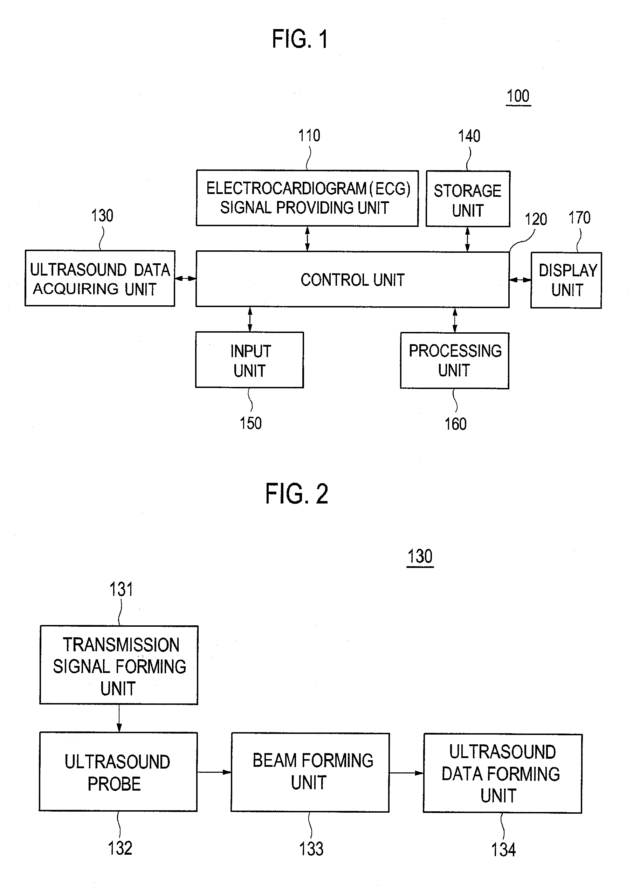

Fig 1 is a diagram illustrating an arrangement of an ultrasound system 100 according to one embodiment of the present invention. The ultrasound system 100 may include an electrocardiogram (ECG) signal providing unit 110, a control unit 120, an ultrasound data acquiring unit 130, a storage unit 140, an input unit 150, a processing unit 160 and a display unit 170.

The ECG signal providing unit 110 may acquire minute electric change resulting from contraction and relaxation of a moving interest object in a target body (e.g., heart, heart muscle, etc.). It may then convert the minute electric change into ECG signals to provide the formed ECG signals.

The control unit 120 may control the synchronization of ultrasound signals by using the ECG signals provided from the ECG signal providing unit 110. In the present embodiment, the control unit 120 may synchronize ultrasound signals with a certain period of the ECG signals (e.g., contraction period (R peak)). The control unit 120 may control transmitting and receiving the synchronized ultrasound signals with the ECG signals. The control unit 120 may control acquiring and storing ultrasound data. The control unit 120 may control storing the acquiring time of the ultrasound data along with the ultrasound data. Further, the control unit 120 may control forming and displaying comparison information. The comparison information will be described below.

The ultrasound data acquiring unit 130 may transmit to the target body the ultrasound signals synchronized with the ECG signals under the control of the control unit 120 and receive from the target body the reflected ultrasound signals (i.e., ultrasound echo signals) to acquire ultrasound data.

Fig. 2 is a block diagram illustrating an arrangement of the ultrasound data acquiring unit 130 according to one embodiment of the present invention. The ultrasound data acquiring unit 130 may include a transmission signal forming unit 131, an ultrasound probe 132 having at least one transducer element, a beam forming unit 133 and an ultrasound data forming unit 134.

The transmission signal forming unit 131 may form transmission signals to be applied to the transducer elements of the ultrasound probe 132 in consideration of the focusing points and the position of the transducer elements. In the present embodiment, the transmission signals may include first transmission signals for acquiring ultrasound data for an object of interest in a target body when the object of interest has not been given medical treatment (hereinafter "first ultrasound data") and second transmission signals for acquiring ultrasound data for the object of interest when the object of interest has been given medical treatment (hereinafter "a second ultrasound data").

The ultrasound probe 132 may transduce the first transmission signals into ultrasound signals when provided with the first transmission signals from the transmission signal forming unit 131. The ultrasound probe 132 may transmit to the target body the ultrasound signals synchronized with ECG signals under the control of the control unit 120 and receive from the target body the reflected ultrasound signals. This is to form first reception signals. The ultrasound probe 132 may transduce the second transmission signals into ultrasound signals when provided with the second transmission signals from transmission signal forming unit 131. The ultrasound probe 132 may transmit to the target body the ultrasound signals synchronized with ECG signals under the control of the control unit 120 and receive from the target body the reflected ultrasound signals. This is to form second reception signals.

The beam forming unit 133 may digitalize the first reception signals to form first digital signals when provided with the first reception signals from the ultrasound probe 132. The beam forming unit 133 may focus the first digital signals to form first focusing reception signals in consideration of the focusing points and the position of the transducer elements of the ultrasound probe 132. Further, the beam forming unit 133 may digitalize the second reception signals to form second digital signals when provided with the second reception signals from the ultrasound probe 132. The beam forming unit 133 may focus the second digital signals to form second focusing reception signals in consideration of the focusing points and the position of the transducer elements of the ultrasound probe 132.

The ultrasound data forming unit 134 may use the first focusing reception signals to form first ultrasound data when provided with the first focusing reception signals from the beam forming unit 133. The ultrasound data forming unit 134 may use the second focusing reception signals to form second ultrasound data when provided with the second focusing reception signals from the beam forming unit 133. Further, the ultrasound data forming unit 134 may perform a variety of signal processes necessary to form ultrasound data (e.g., gain control, filtering control, etc.) from the first and second focusing reception signals.

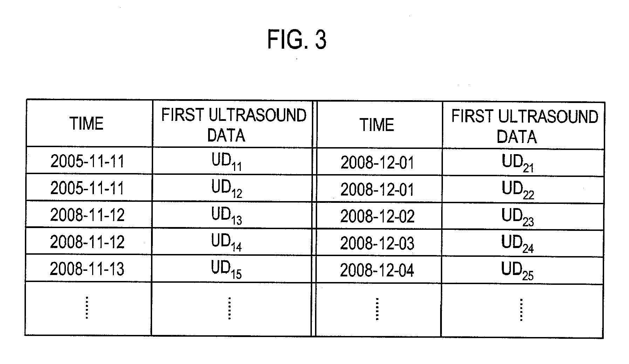

Referring back to Fig. 1, the storage unit 140 may store the first and second ultrasound data provided from the ultrasound data acquiring unit 130. For example, the storage unit 140 may store the first ultrasound data and the second ultrasound data by order of the acquiring time of the ultrasound data under the control of the control unit 120, as illustrated in Fig. 3.

The input unit 150 may be a control panel, a mouse, a keyboard and the like, which may receive instructions from a user. In the present embodiment, the instructions may include the instructions for selecting at least one of a plurality of the first ultrasound data stored in the storage unit 140 and at least one of a plurality of the second ultrasound data stored in the storage unit 140.

The processing unit 160 may look up the storage unit 140 and extract from the storage unit 140 the first ultrasound data and the second ultrasound data when provided with the instructions from the input unit 150. The processing unit 160 may compare the extracted first ultrasound data and the extracted second ultrasound data to form comparison information. For example, when provided with the instructions for selecting first ultrasound data UD 14 and second ultrasound data UD23 from the input unit 150, the processing unit 160 may look up the storage unit 140 and extract the first ultrasound data UD14 and the second ultrasound data UD23. Then, the processing unit 160 may compare the extracted first ultrasound data UD14 and the extracted second ultrasound data UD23 to form comparison information. For the sake of the brevity, detailed explanations on the conventional techniques of forming comparison information between ultrasound data are not provided herein. Further, the processing unit 160 may form a list of the first and second ultrasound data, which are stored in the storage unit 140.

The display unit 170 may display the comparison information provided from the processing unit 160. Further, the display unit 170 may display a list of the first and second ultrasound data, which is provided from the processing unit 160.

According to the present invention, it is easier and more convenient to identify how much an object of interest is improved through a medical treatment by comparing ultrasound data associated with the object of interest before and after the medical treatment and providing comparison information.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art. The invention is not to be restricted except in the spirit of the appended claims.

## Claims

1. An ultrasound system, comprising:
an ultrasound data acquisition unit configured to acquire a plurality of first ultrasound data for an object of interest in a target body when the object of interest is in a first state and acquire a plurality of second ultrasound data for the object of interest when the object of interest is in a second state;
a storage unit for storing the plurality of first ultrasound data and the plurality of second ultrasound data;
an input unit configured to receive a user instruction for selecting at least one of the plurality of first ultrasound data and at least one of the plurality of second ultrasound data; and
a processing unit configured to extract the at least one of the plurality of first ultrasound data and the at least one of the plurality of second ultrasound data from the storage unit and compare the extracted first ultrasound data with the extracted second ultrasound data to form comparison information.

2. The system of Claim 1, wherein the object of interest has not been given medical treatment in the first state and the object of interest has been given medical treatment in the second state.

3. The system of Claim 1, wherein the ultrasound data acquisition unit is configured to transmit an ultrasound signal to the target body and receive the ultrasound signal reflected from the target body to acquire the first and second ultrasound data.

4. The system of Claim 3, further comprising:
an ECG signal providing unit configured to provide an electrocardiogram (ECG) signal of the object of interest; and
a control unit configured to synchronize the ultrasound signal with the ECG signal.

5. The system of Claim 1, further comprising:
a display unit configured to display the comparison information.

6. An ultrasound system, comprising:
an ultrasound data acquisition unit configured to acquire first ultrasound data for an object of interest in a target body without medical treatment given and
acquire second ultrasound data for the object of interest with medical treatment given; and
a processing unit configured to compare the first ultrasound data with the second ultrasound data to form comparison information.

7. A method of providing comparison information, comprising:
a) acquiring a plurality of first ultrasound data for an object of interest in a target body when the object of interest is in a first state and a plurality of second ultrasound data for the object of interest in the target body when the object of interest is in a second state;
b) storing in a storage unit the plurality of first ultrasound data and the plurality of second ultrasound data;
c) receiving a user instruction for selecting at least one of the plurality of first ultrasound data and at least one of the plurality of second ultrasound data;
d) extracting the at least one of the plurality of first ultrasound data and the at least one of the plurality of second ultrasound data from the storage unit; and
e) comparing the extracted first ultrasound data with the extracted second ultrasound data to form comparison information.

8. The method of Claim 7, wherein the object of interest has not been given medical treatment in the first state and the object of interest has been given medical treatment in the second state.

9. The method of Claim 7, wherein a) comprises:
transmitting an ultrasound signal to the target body and receiving the ultrasound signal reflected from the target body to acquire the first and second ultrasound data.

10. The method of Claim 9, further comprising:
providing an electrocardiogram (ECG) signal of the object of interest; and
synchronizing the ultrasound signal with the ECG signal.

11. The method of Claim 7, further comprising:
displaying the comparison information.
